# FASCICULE DE BREVET EUROPEEN

(11) **EP 1 124 986 B1**
(45) Date de publication et mention de la délivrance du brevet: **06.05.2004**
(21) Numéro de dépôt: 99971865.3
(22) Date de dépôt: 05.11.1999
(51) Int. Cl.: C12Q 1/04, C07D 311/16, C12Q 1/26

(54) **NITROCOUMARINES POUR LA DETECTION DE TOUS MICRO-ORGANISMES**
NITROCUMARINE FÜR DEN NACHWEIS ALLER MIKROORGANISMEN
NITROCOUMARINS FOR DETECTING ALL MICRO-ORGANISMS

(30) Priorité: 05.11.1998 FR 9814101
(43) Date de publication de la demande: 22.08.2001
(73) Titulaire: Biomerieux S.A., 69280 Marcy l'Etoile (FR)
(72) Inventeur: JAMES, Arthur, Jesmond Newcastle-upon-Tyne NE2 1HR (GB); MONGET, Daniel, 01150 Saint Sorlin en Bugey (FR)
(74) Mandataire: Bonneau, Gérard
(86) Numéro de dépôt international: PCT/FR1999/002704
(87) Numéro de publication internationale: WO 2000/028073

(56) Documents cités:
- EP-A- 0 397 362
- US-A- 4 728 608
- HISASHI ICHIBAGASE: "Syntheses of coumarin derivatives. V. Syntheses of coumarin-3-carboxylic acid derivatives." CHEMICAL ABSTRACTS, vol. 50, 1956, page 10715 XP002109446 cité dans la demande & J. PHARM. SOC. JAPAN, vol. 75, 1955, pages 1477-1480,
- MASATAKA ICHIKAWA, HISASHI ICHIBAGASE: "Syntheses of coumarin derivatives. XIV. Preparation of 5-hydroxy-7-nitro-3-coumarincarboxylic acid" CHEMICAL ABSTRACTS, vol. 59, 1963, page 2757 XP002109447 cité dans la demande & PHARMACEUTICAL SOCIETY OF JAPAN. JOURNAL - YAKUGAKU ZASSHI, vol. 83, 1963, pages 103-106,
- M ICHIKAWA, H ICHIBAGASE: "Studies on Synthesis of Coumarin Derivatives. XX. Synthesis and Antibacterial Activity of Derivatives of N-Substituted 3-Coumarincarboxamide" CHEMICAL AND PHARMACEUTICAL BULLETIN, vol. 16, no. 11, novembre 1968 (1968-11), pages 2093-2100, XP002109448 cité dans la demande
- H SAIKACHI, M ICHIKAWA: "Synthesis of Coumarin Derivatives. XV. On the Preparation of Ethyl Pyranobenzoxazole carboxylates." CHEMICAL AND PHARMACEUTICAL BULLETIN, vol. 14, 1966, pages 1162-1167, XP002109449 cité dans la demande
- SPAIN JC: "Biodegradation of nitroaromatic compounds" ANNUAL REVIEW OF MICROBIOLOGY, vol. 49, 1995, pages 523-555, XP002109450
- RAFII, FATEMEH; FRANKLIN, WIRT; HEFLICH, ROBERT H.; CERNIGLIA, CARL E.: "Reduction of nitroaromatic compounds by anaerobic bacteria isolated from the human gastrointestinal tract" APPLIED AND ENVIRONMENTAL MICROBIOLOGY, vol. 57, no. 4, 1991, pages 962-968, XP002109451
- BRYANT C, DELUCA M: "Purification and characterization of an oxygen-insensitive NAD(P)H nitroreductase from Enterobacter cloacae" JOURNAL OF BIOLOGICAL CHEMISTRY, vol. 266, no. 7, 5 mars 1991 (1991-03-05), pages 4119-4125, XP002109452
- RIEBLE S, JOSHI DK, GOLD MH: "Aromatic nitroreductase from the basidiomycete Phanerochaete chrysosporium" BIOCHEMICAL AND BIOPHYSICAL RESEARCH COMMUNICATIONS, vol. 205, no. 1, 30 novembre 1994 (1994-11-30), pages 298-304, XP002109453

## Description

La présente invention concerne un composé nitro-aromatique capable d'être réduit en un produit amino-aromatique par des micro-organismes.

L'invention propose également l'utilisation d'un composé dans un test de détection et/ou de diagnostic de micro-organismes.

Ladite invention a encore pour objet un procédé de mise en évidence d'une activité nitroaryl-réductase dans un milieu de culture de micro-organismes.

L'invention a enfin trait à un procédé de détection d'un micro-organisme ou d'un groupe de micro-organismes dans un échantillon susceptible de les contenir.

*L'article « Syntheses of coumarin derivatives. V. Syntheses of coumarin-3-carboxylic acid derivatives. » CHEMICAL ABSTRACTS, vol. 50, 1956, page 10715, XP-002109446, décrit la synthèse de dérivés de nitrocoumarines et même de 7-nitrocoumarines. La seule application qui soit décrite concerne des actions hypnotique et sédative. De plus, des études de toxicité ont été réalisées. L'article* « *Syntheses of coumarin derivatives. XIV. Preparation of 5-hydroxy-7-nitro-3-coumarincarboxylic acid » CHEMICAL ABSTRACTS, vol. 59, 1963, page 2757, XP-002109447, ne décrit quant à lui que la synthèse de l'acide 5-hydroxy-7-nitrocoumarine carboxylique.*

L'utilisation de ces molécules dans la détection de la présence ou de l'absence de micro-organismes n'est jamais abordée. Il n'y a donc aucune application à la bactériologie qui soit envisagée. De plus, l'étude de toxicité n'engage pas l'homme du métier à penser pouvoir utiliser de tels composés dans des milieux de culture pour la bactériologie qui permettent la croissance de micro-organismes.

*Certains produits synthétisés ci-dessus peuvent avoir une application à des fins thérapeutiques, certains en particulier pouvant avoir des propriétés antibactériennes. C'est ce que révèle les deux articles suivants. Ainsi, l'article « Studies on Synthesis of Coumarin Derivatives. XX. Synthesis and Antibacterial Activity of Derivatives of N-Substituted 3-Coumarincarboxamide » CHEMICAL AND PHARMACEUTICAL BULLETIN, vol. 16, no. 11, novembre 1968 (1968-11) pages 2093-2100. XP* *002109448, décrit l'utilisation de nitrocoumarines dans des tests antibactériens, ce qui est également le cas de l'article « Synthesis of Coumarin Derivatives. XV. On the Preparation of Ethyl Pyranobenzoxazole carboxylates. » CHEMICAL AND PHARMACEUTICAL BULLETIN, vol. 14, 1966, pages 1162-1167, XP-002109449.*

Néanmoins dans notre invention, la propriété essentielle qui nous intéresse est la possibilité pour les nitrocoumarines et plus particulièrement les 7-nitrocoumarines d'être fluorescentes à l'état réduit. Cette fluorescence est caractéristique de la présence ou de l'absence de certains micro-organismes. Or la toxicité et les propriétés antibactériennes ne peuvent que détourner l'homme du métier de toutes velléités d'utiliser de telles molécules pour détecter des micro-organismes.

L'objet de notre demande de brevet est tout à fait différent. Il ne s'agit en aucun cas de rechercher une activité thérapeutique, comme l'inhibition de la croissance bactérienne, mais au contraire de mettre en évidence des micro-organismes par la recherche d'une activité enzymatique de type nitro-réductase qui réduit la 7-nitrocoumarine non fluorescente (ou dérivé) en 7-aminocoumarine fluorescente (ou dérivé). Il s'agit donc d'un test fluorescent de détection universelle de micro-organismes dans un échantillon susceptible de les contenir, où il n'y a aucun intérêt à avoir une inhibition bactérienne.

*La capacité de certaines bactéries à réduire les composés nitro-aromatiques est connue depuis de nombreuses années. Asnis (1957) a rapporté l'isolation d'une flavoprotéine à partir d'extraits d'E. coli qui était capable de réduire l'acide p-nitrobenzoïque. Depuis ce rapport, l'activité nitroaryl-réduclase a été identifiée dans diverses variétés d'organismes. Ceci inclut des aérobies strictes tels que Pseudomonas spp. (Won et al. 1974) et Nocardia spp. (Villanueva 1964), des anaérobies strictes tels que Clostridium spp. (Ancermaier & Simon 1983) et Veillonella spp. (McCormick et al. 1976), ou encore des champignons (Masuda & Ozaki 1993) et des parasites eucaryotes (Douch 1975). Il existe une gamme de substrats qui ont été désignés comme étant susceptibles d'être réduits par des nitroaryl-réductases bactériennes. Il s'agit surtout de composés nitro-aromatiques tels que l'acide p-nitrobenzoïque, le p-nitrophénol, la p-nitroaniline et le 2, 4, 6-trinitrotoluène (McCormick et al. 1976).*

Bien qu'une grande diversité de substrats soit disponible, aucun ne permet la détection directe de nitroaryl-réductases, en donnant un produit fluorescent.

*La détection de l'activité enzymatique doit donc être réalisée par des méthodes indirectes telles que le suivi de la disparition du substrat ou d'un cofacteur. Kitamura et al. (1983) ont étudié la réduction du méthyl p-nitrobenzoate et d'une gamme d'autres composés aromatiques nitrés avec des extraits d'E. coli. Ils ont montré que trois activités enzymatiques distinctes pouvaient être isolées en utilisant la chromatographie avec une colonne DEAE-cellulose et que les trois fractions bien définies avaient des besoins différents en cofacteur pour leur activité. La première est liée à la présence de NADH, la seconde à la présence de NADPH, et la troisième à la présence d'un des deux. La réactivité de ces enzymes a été mesurée en contrôlant la diminution de densité optique (D.O.) à 340 nanomètre (nm), corrélée à la disparition de NADH et*/*ou de NADPH. La diminution de NADH et*/*ou NADPH a été associée à la formation de deux produits de réaction, le méthyl p-aminobenzoate et le méthyl p-hydroxylaminobenzoate. Bryant et al. (1981) ont également étudié les nitroaryl-réductases de E. coli en utilisant la nitrofurazone comme substrat. Pour mesurer l'activité enzymatique, ils ont pu contrôler la diminution de D.O. à 375 nm, qui correspond à la longueur d'onde maximale (λ max.) de la nitrofurazone. En utilisant cette méthode, ils ont pu mettre en évidence trois activités enzymatiques distinctes capables de réduire la nitrofurazone. La capacité qu'ont les bactéries de réduire les nitrofuranes est d'un intérêt considérable en chimiothérapie antimicrobienne (Peterson et al. 1979, Wentzell & McCalla 1980). En effet, il a été montré que l'activité nitroaryl-réductase majeure chez E. coli, qui est uniquement dépendante de la présence de NADPH comme cofacteur, est absente chez les mutants résistants à la nitrofurazone (Bryant et al. 1981).*

La présente invention a pour objet un substrat fluorogène à base de nitrocoumarine pour la détection directe des activités nitroaryl-réductases. Ce type de composé nitro-aromatique est capable de produire, après réduction, un composé très fluorescent qui est donc facilement détectable. Cette réaction 1 est la suivante :

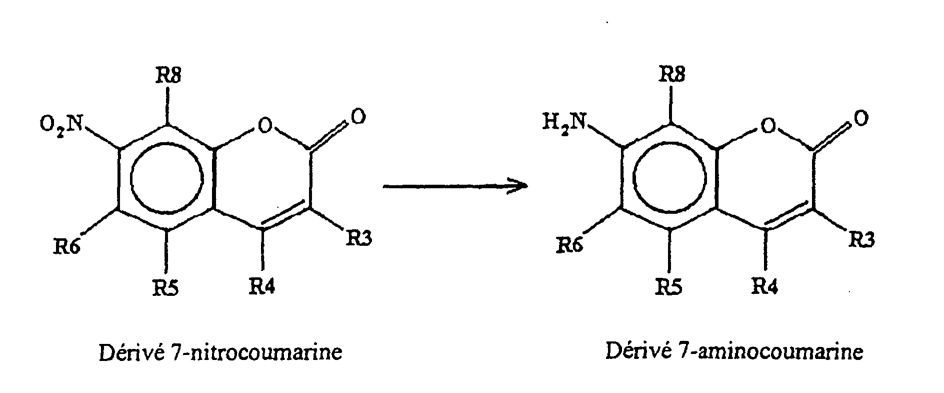

De façon très surprenante, il a été constaté que la très grande majorité des micro-organismes sont capables, grâce à leurs activités nitroréductases, de réduire les dérivés de la 7-nitrocoumarine en composés fluorescents, ce qui n'est pas le cas avec les substrats connus à ce jour. Avec ces dérivés, il existe maintenant une famille d'indicateurs universels permettant la détection de la présence ou de l'absence de micro-organismes dans un échantillon donné.

A cet effet, la présente invention concerne un composé de détection de la présence ou de l'absence d'au moins un micro-organisme, caractérisé par le fait que le composé est constitué par une molécule de nitrocoumarine ou un des ses dérivés, qui est fluorescent à l'état réduit.

Préférentiellement, ledit composé est constitué par une molécule de 7-nitrocoumarine ou un de ses dérivés.

Le composé a la caractéristique d'être constitué par une molécule ayant la formule générale suivante : 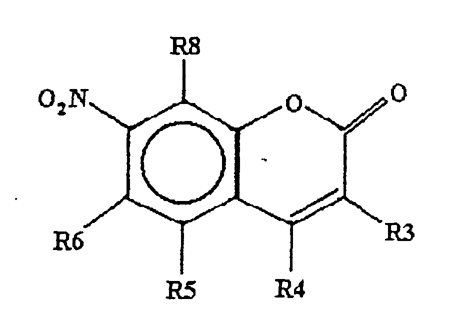 dans laquelle R₃ est choisi parmi : H ou COZ, où Z permet la formation d'une cétone, d'un acide, d'un ester ou de tout autre composé aliphatique, et dans laquelle R₄ est choisi parmi : H ou trifluorométhyle (CF₃) ou tout composé aliphatique.

Selon une variante, R₃ est constitué par : COOCH₃, COOC₂H₅, COOH, COC₃H₇, CONC₄H₄O ou COCH₃, et R₄ est constitué par : H ou CH₃.

Selon une autre variante, le composé est constitué par de l'acide 7-nitrocoumarine-3-carboxylique.

La concentration en acide 7-nitrocoumarine-3-carboxylique est comprise entre 0,05 et 0,3 mmol/l.

Selon un mode de réalisation intéressant, le composé décrit ci-dessus peut être utilisé dans une composition en combinaison avec au moins une autre molécule de nitrocoumarine ou un des ses dérivés.

L'invention concerne également l'utilisation d'un composé, tel que défini ci-dessus, dans un test de détection et/ou de diagnostic de la présence ou de l'absence de micro-organismes.

L'invention concerne encore un premier procédé de détection d'un micro-organisme ou d'un groupe de micro-organismes dans un échantillon susceptible de les contenir, comprenant les étapes consistant à :
- ajouter à un milieu de culture, contenant l'échantillon, au moins un composé, à base de nitrocoumarine et préférentiellement de 7-nitrocoumarine ou un de leurs dérivés, et
- rechercher la formation d'un produit fluorescent, la présence ou l'absence de cette fluorescence permet de conclure respectivement à la présence ou à l'absence du micro-organisme ou du groupe de micro-organismes recherché.

L'invention concerne aussi un second procédé d'identification d'au moins un micro-organisme dans un échantillon susceptible de les contenir comprenant les étapes suivantes :
- ajouter dans différents puits d'identification un milieu de culture, contenant chacun une seule source de carbone, telle que lactose, glucose, saccharose... , une fraction de l'échantillon à analyser et au moins un composé, à base de nitrocoumarine et préférentiellement de 7-nitrocoumarine ou un de leurs dérivés, et
- rechercher la formation d'un produit fluorescent, dans chaque puits, la présence ou l'absence de cette fluorescence sur l'ensemble des puits permet d'identifier le micro-organisme. Ce second procédé est également appelé test d'assimilation.

Enfin, l'invention concerne différentes applications associées à la détection de la croissance microbienne à l'aide d'au moins un composé, tel que défini ci-dessus, pour :
- effectuer un contrôle de stérilité,
- effectuer une numération des micro-organismes présents dans l'échantillon,
- déterminer la sensibilité d'un micro-organisme aux agents antimicrobiens, et
- détecter la présence d'au moins un micro-organisme.

Les figures ci-jointes sont données à titre d'exemple explicatif et n'ont aucun caractère limitatif. Elles permettront de mieux comprendre l'invention.

La figure 1 représente un graphique montrant l'effet d'une gamme de nitrocoumarines à 0,105 mmol/l sur la croissance d'*E*. *coli* (NCTC 10418) dans un bouillon Mueller-Hinton.

La figure 2 représente un graphique montrant la fluorescence générée par *E. coli* (NCTC 10418) en présence de différents dérivés de nitrocoumarines à 0,105 mmol/l dans un bouillon Mueller-Hinton.

La figure 3 représente un graphique montrant la croissance d'*E*. *coli* (NCTC 10418) en présence de concentrations variables d'acide 7-nitrocoumarine-3-carboxylique dans un bouillon Mueller-Hinton.

La figure 4 représente un graphique montrant la fluorescence générée par *E. coli* (NCTC 10418) en présence de différentes concentrations d'acide 7-nitrocoumarine-3-carboxylique dans un bouillon Mueller-Hinton.

La figure 5 représente un graphique montrant la croissance de différents souches sauvages d'*Enterobacteriaceae* dans un bouillon Mueller-Hinton en présence d'acide 7-nitrocoumarine-3-carboxylique à 0,17 mmol/l.

La figure 6 représente un graphique montrant la réduction de l'acide 7-nitrocoumarine-3-carboxylique à 0,17 mmol/l par les souches sauvages d'*Enterobacteriaceae* de la figure 5.

La présente invention concerne une gamme de composés basés sur la 7-nitrocoumarine, qui constitue un substrat fluorogène pour la détection directe des activités nitroaryl-réductases.

La structure générale des nitrocoumarines est représentée par la formule II :

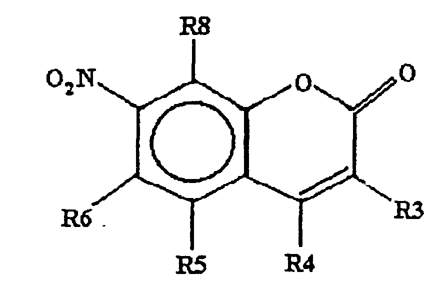

La liste des dérivés substitués est assez importante. Ainsi les expérimentations ont portées sur chacune des substitutions et sur les différentes possibles. Cette liste est représentée dans le tableau 1 ci-après. Elle concerne essentiellement les radicaux R₃, R₄ et R₈, qui sont les substituants les plus importants.

Néanmoins les radicaux R₅ et R₆ sont généralement non substitués comme cela est le cas dans le tableau 1. Ils sont alors constitués par des atomes d'hydrogène (H). Pourtant, ils peuvent être différents et être constitués par :
- au moins l'un des radicaux R₅ et R₆ est constitué par CH₃ ou un alkyle de petite taille (nombre d'atomes de carbone inférieur à 5), ou
- au moins l'un des radicaux R₅ et R₆ est constitué par un halogénure (F, Cl, Br, I), ou
- au moins l'un des radicaux R₅ et R₆ est constitué par CH₃O ou un alkoxy de petite taille (nombre d'atomes de carbone inférieur à 5), ou
- au moins l'un des radicaux R₅ et R₆ est constitué par un phényle (aryle) ou aralkyle. Dans le cas où seul un des radicaux R₅ ou R₆ est l'un des groupements ci-dessus énuméré, l'autre radical est constitué par H.

De plus il est possible que R₅ et R₆ forment un cycle aromatique (benzenoïde ou hétérocyclique). La structure d'une telle molécule est décrite ci-contre.

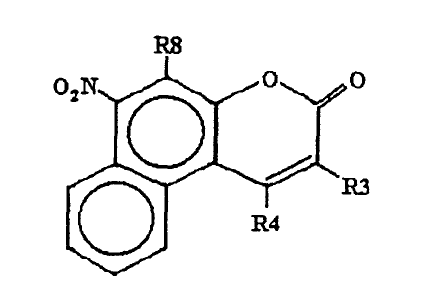

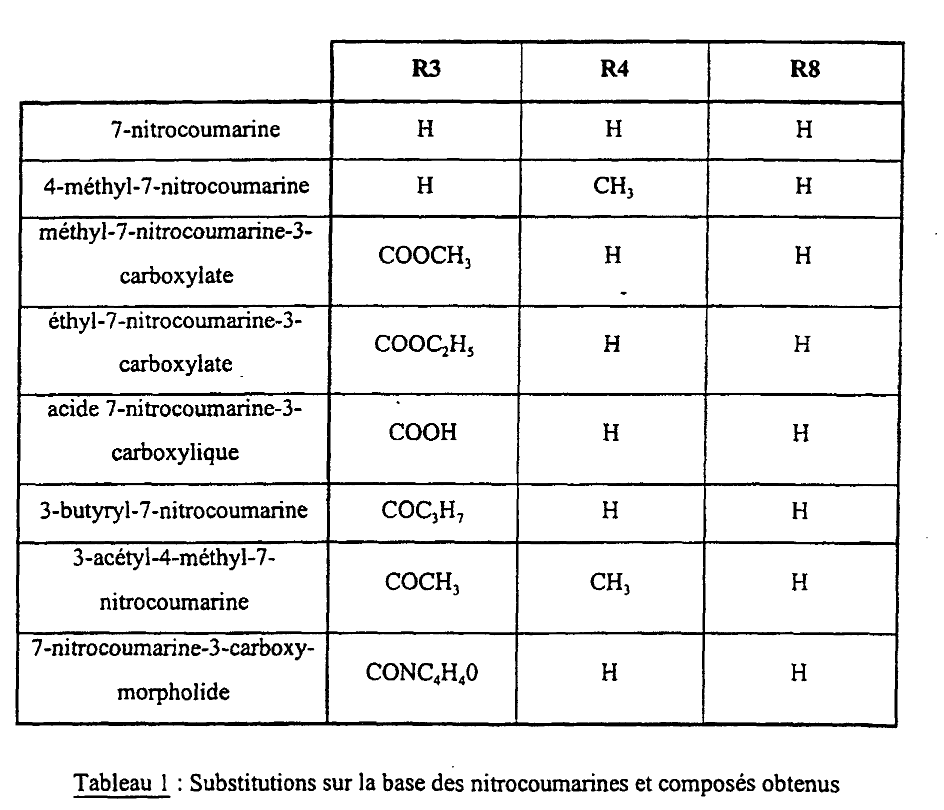

### 1°) Matériels :

### A - Milieu de croissance :

Les milieux utilisés sont constitués par des bouillons et géloses Mueller-Hinton et Trypticase Soja, qui ont été obtenus chez Unipath Ltd, Basingstoke, Grande Bretagne.

### B - Substrats et produits chimiques :

Les substrats suivants ont été synthétisés : 7-nitrocoumarine, 4-méthyl-7-nitrocoumarine, méthyl-7-nitrocoumarine-3-carboxylate, éthyl-7-nitrocoumarine-3-carboxylate, acide 7-nitrocoumarine 3-carboxylique, 3-butyryl-7-nitrocoumarine, 3-acétyl-4-méthyl-7-nitrocoumarine, 7-nitrocoumarine-3-carboxy-morpholide.

### C - Equipement :

L'équipement utilisé a été le suivant :
- lecteur spectrophotométrique de plaques de microtitration Anthos 2001, en provenance de chez Labtech International Limited, Uckfield, Grande Bretagne, et
- lecteur à fluorescence de plaques de microtitration Labtech Biolite F1, en provenance de chez Labtech International Limited, Uckfield, Grande Bretagne.

### D - Synthèse des nitrocoumarines :

Les produits chimiques impliqués dans la synthèse des nitrocoumarines ont tous été obtenus auprès de Aldrich Chemical Company Ltd, Gillingham, Grande-Bretagne.

La synthèse de la 7-nitrocoumarine a été décrite par LIEBERMANN, M, et al. (1951) *Académie des Sciences* **232**, 2027-2029. Elle consiste à chauffer à reflux, pendant 3 h, 12 g d'aldéhyde nitro-4-salicylique, 18 g d'acétate de sodium anhydre et 27 g d'anhydride acétique. On coule dans un mortier et on réduit la masse en pâte, puis on essore et on lave avec de petites quantités d'anhydride acétique, et ensuite à l'eau. Le produit est chauffé 2 h à reflux en présence de 13 g de CO₃Na₂ et 320 cm³ d'eau. On filtre à chaud et on précipite également à chaud par l'acide chlorhydrique. On essore après refroidissement et on recristallise dans 300 cm³ d'acide acétique à 50%. Une nouvelle cristallisation donne un produit fondant à 198-200°C.

La synthèse de différents dérivés de nitrocoumarine nécessite tout d'abord la synthèse de 4-nitrosalicylaldéhyde, qui a été produit en utilisant une modification de la méthode décrite par SEGESSER, J.R., et CALVIN, M. (1942) *J. Am. Chem. Soc*. **64**, 825-826. Ceci implique une acétylation initiale de la 2-méthyl-5-nitrophénol suivie par deux étapes d'ajout de brome, utilisant du N-bromosuccinimide et un catalyseur de péroxyde de benzoyle dans un solvant de tétrachlorure de carbone pour obtenir du 2-acétoxy-4-nitrobenzale bromure. Le dibromure brut est recristallisé à partir de 1-butanol et converti en du nitrosalicylaldéhyde par la procédure suivante.

Un échantillon de 11 grammes (g) de dibromure purifié est dissout dans 50 millilitres (ml) de méthanol sec et l'ensemble est ajouté graduellement à 250 ml d'une solution en ébullition de sodium dans du méthanol (1 % w/v). Après reflux pendant 45 minutes (mn), la solution orange foncée est refroidie et une quantité de100 ml d'eau est ajoutée. Après ébullition pendant encore 15 mn, la solution est refroidie et son pH est amené à une valeur de 3. Le méthanol est retiré par une évaporation rotative et le précipité de 4-nitrosalicylaldéhyde est séparé par filtration aspirante. Le restant est alors recristallisé dans de l'éthanol dilué.

Trois des dérivés de nitrocoumarine ont été synthétisés par la même méthode qui implique une condensation de Knoevenagel avec du 4-nitrosalicylaldéhyde. Du diméthylmalonate (1,45 g, 11 mM) et 4-nitrosalicylaldéhyde (1,67 g, 10 mM) sont dissous dans 15 ml d'éthanol. Une masse de 100 mg de pipéridine et un volume de 100 µl de d'acide acétique froid sont alors ajoutés et le mélange est reflué pendant 2 h. Le méthyl-7-nitrocoumarine-3-carboxylate est formé soit par séparation pendant la réaction soit par cristallisation pendant le refroidissement. Le produit est retiré par filtration aspirante et recristallisé dans de l'éthanol liquide. Pour la synthèse de l'éthyl-7-nitrocoumarine-3-carboxylate et du 3-butyryl-7-nitrocoumarine, le diméthyl malonate est remplacé respectivement soit par du diéthyl malonate, soit par de l'éthylbutyrylacétate.

L'acide 7-nitrocoumarine-3-carboxylique est obtenu par refluage de l'éthyl 7-nitrocoumarine-3-carboxylate (2,63 g, 10 mM) avec un excès de solution d'hydroxyde de potassium et d'éthanol aqueux pendant 1 h. La solution de couleur jaune profonde du sel de potassium est alors acidifiée à l'aide d'acide chlorydrique et le produit récolté, lavé avec une petite quantité d'eau, est ensuite recristallisé à partir d'eau en ébullition.

La 7-nitrocoumarine-3-carboxymorpholide est préparée comme suit. L'acide 7-nitrocoumarine-3-carboxylique (1,17 g, 5 mM) est dissout dans un mélange de 25 ml de tétrahydrofurane anhydre et de 10 ml de diméthylformamide. A cette solution, préalablement mélangée, est ajouté 5 mmol de N-méthylmorpholine (0,5 g, 5 mM) et, après refroidissement à une température de moins 12 degrés Celsius (° C), est ajouté de l'isobutyle chloroformate (0,68 g, 5 mM). Après 10 mn, le morpholide (0,64 g, 7,5 mM) est ajouté. La réaction peut être réalisée pendant 30 mn, au 0 absolu de température, suivie par une étape de 5 h à la température ambiante. L'hydrochlorure de N-méthylmorpholine est retiré par filtration et le filtrat est versé dans 10 ml d'un mélange eau/glace. Le solide, qui est séparé, est recristallisé dans du méthanol liquide.

La synthèse à la fois du 4-méthyl-7-nitrocoumarine et du 3-acétyl-4-méthyl-7-nitrocoumarine est effectuée par une procédure alternative qui implique l'oxydation de la 7-aminocoumarine. La 7-amino-4-méthylcoumarine (1,75 g, 10 mM) est suspendue dans 10 ml d'acide sulfurique (75 % w/w) et l'ensemble est mélangé efficacement. Une quantité de 2,5 ml de solution de nitrite de sodium (7 M) est ajouté graduellement à moins 5 °C, au moyen d'une longue colonne de refoulement qui atteint le fond du tube de réaction. La solution de diazonium est mélangée pendant 15 mn à moins 2° C jusqu'à atteindre 5 °C. Un échantillon de 5 l d'eau glacée contenant 2,4 g de tétrafluoroborate de sodium est ajouté lentement à la solution de diazonium froid. L'eau glacée est ajoutée lentement à 0 °C jusqu'à ce que le volume forme une masse de cristaux de tétrafluoroborate. Cette substance est retirée par filtration aspirante et les cristaux sont nettoyés avec un peu d'eau glacée, nettoyage qui se poursuit avec du méthanol et finalement avec de l'éther. Le produit est rapidement séché à l'air sec générant ainsi 2,2 g de produit.

Une masse de 3 g d'échantillon de poudrè de cuivre et une suspension d'oxyde cuivreux (préparé par réduction glucosée de sulfate cuivreux) sont ajoutées à une solution refroidie de nitrite de sodium (4,1 M). Un échantillon de 2,2 g de sel de diazonium est suspendu dans 10 ml d'eau et ajouté en plusieurs aliquotes durant 20 mn à une température comprise entre 5 et 15 °C sous agitation. L'azote est retiré ce qui nécessite l'addition d'une petite quantité d'éther pour prévenir l'apparition de mousse. Le mélange se poursuit pendant 5 h. La suspension est filtrée, nettoyée avec de l'eau et ensuite extraite avec de l'acétate d'éthyle chaud. La solution aqueuse est pareillement extraite. Les extraits combinés sont nettoyés avec de l'eau et séchés avec du sulfate de magnésium anhydre. Un solvant est retiré par évaporation rotative pour donner une résidu jaune. La recristallisation à partir d'acide acétique chaud, donne 0,62 g de produit jaune citron, c'est-à-dire de 4-méthyl-7-nitrocoumarine.

La 3-acétyl-4-méthyl-7-nitrocoumarine est préparée par une méthode similaire utilisant le 3-acétyl-4-méthyl-aminocoumarine. Le dernier composé est préparé par une modification de la méthode de synthèse du 7-amino-4-méthylcoumarine, qui consiste en une substitution de l'éthyle diacétoacétate par de l'éthyle acétoacétate.

### E - Préparation des solutions de substrat :

Un échantillon de dérivé de 7-nitrocoumarine est dissout à chaud dans 4 ml d'eau distillée. La quantité dissoute est déterminée de façon à obtenir une concentration finale de 0,105 mmol/l lors de l'essai. Cette solution est ensuite ajoutée à 96 ml de bouillon Mueller-Hinton, et le mélange est filtré de manière stérile.

### F - Micro-organismes étudiés :

Les micro-organismes utilisés sont soit des souches sauvages, soit des souches provenant de collections internationales (NCTC, ATCC)

### 2°) Méthodes et résultats :

### A - Evaluation de la valeur de différents dérivés de nitrocoumarine comme indicateurs de croissance de souches d'Escherichia coli (NCTC 10418) :

### 1°) Méthode :

Une première étude a été réalisée à l'aide de la souche d' *Escherichia coli* (NCTC 10418) et les huit nitrocoumarines décrites structurellement dans le tableau 1, pour mémoire, il s'agit de :
- 7-nitrocoumarine,
- 4-méthyl-7-nitrocoumarine,
- méthyl-7-nitrocoumarine-3-carboxylate,
- éthyl-7-nitrocoumarine-3-carboxylate,
- acide 7-nitrocoumarine 3-carboxylique,
- 3-butyryl-7-nitrocoumarine,
- 3-acétyl-4-méthyl-7-nitrocoumarine, et
- 7-nitrocoumarine-3-carboxymorpholide.

La souche d'*E.coli* est cultivée à 35°C sur gélose Columbia au sang de mouton pendant 24 h. Une suspension est ensuite réalisée dans de l'eau distillée stérile et ajustée au point 0,5 de l'échelle de Mac Farland, soit environ 10⁸ cellules par millilitre (cellules/ml), puis diluée au centième dans du bouillon Mueller-Hinton stérile, soit environ 10⁶ (cellules/ml).

Cinquante microlitres de chaque solution de nitrocoumarines et cinquante microlitres de suspension microbienne sont ajoutés dans les puits d'une plaque de microtitration. Les plaques ainsi préparées sont mises à incuber à 35°C et lues toutes les 30 mn pendant 4 h - en densité optique (690 nm), à l'aide du spectrophotomètre Anthos 2001 (Labtech International Limited), et en fluorescence (excitation : 365 nm et émission : 440 nm), à l'aide du fluorimètre Biolite F1 2001 (Labtech International Limited).

### 2°) Résultats :

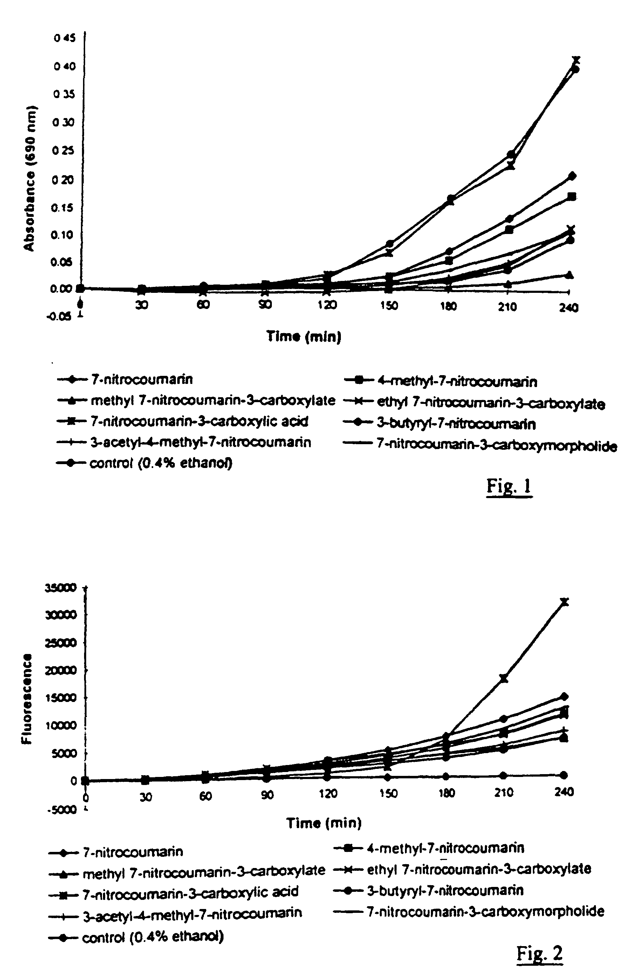

La figure 1 montre l'effet de chaqcune des huit nitrocoumarines (à 0,105 mmol/l) sur la croissance d'*E. coli* (NCTC 10418). On voit clairement sur cette figure que la substitution chimique du noyau coumarine a un effet significatif sur les caractéristiques inhibitrices de la 7-nitrocoumarine.Par exemple, en présence de 0,105 mmol/l d'acide 7-nitrocoumarine-3-carboxylique, il n'y a pas de mise en évidence d'inhibition de croissance. Toutefois, en présence de 0,105 mmol/l de méthyl-7-nitrocoumarine-3-carboxylate, la D.O. globale est réduite de 92% par comparaison avec le contrôle de croissance.Ces effets inhibiteurs reflètent largement le taux de réduction de substrat tel qu'il a été mesuré par production de fluorescence.

On voit clairement sur la figure 2, que les deux composés les plus inhibiteurs, le méthyl-7-nitrocoumarine-3-carboxylate et la 3-butyryl-7-nitrocoumarine, sont les moins réduits après la période de quatre heures. De plus, la réduction de l'acide 7-nitrocoumarine-3-carboxylique, seul composé non inhibiteur, a largement dépassé celle des autres nitrocoumarines et la fluorescence générée est plus de deux fois supérieure à celle produite par n'importe quel autre substrat.

Pour faciliter la lecture des figures 1 et 2, les deux tableaux 2 et 3 qui suivent énumèrent les valeurs associées à ces figures.

**Tableau 2 :**

| Valeurs numériques associées à la figure 1 | | | | | | | | | |
|---|---|---|---|---|---|---|---|---|---|
| **Composé\Temps(mn)** | **0** | **30** | **60** | **90** | **120** | **150** | **180** | **210** | **240** |
| **7-nitrocoumarine** | 0.000 | -0,002 | 0,000 | 0,002 | 0,009 | 0,025 | 0,072 | 0,133 | 0,211 |
| **4-méthyl-7-nitrocoumarine** | 0,000 | 0,000 | 0,002 | 0,004 | 0,010 | 0,024 | 0,055 | 0,113 | 0,175 |
| **méthyl-7-nitrocoumarine-3-carboxylate** | 0,000 | -0,001 | -0,001 | 0,000 | 0,000 | 0,002 | 0,006 | 0,014 | 0,032 |
| **éthyl-7-nitrocoumarine-3-carboxylate** | 0,000 | -0,005 | -0,008 | -0,008 | -0,006 | 0,000 | 0,018 | 0,046 | 0,116 |
| **acide 7-nitrocoumarine 3-carboxylique** | 0,000 | 0,000 | 0,002 | 0,008 | 0,026 | 0,068 | 0,164 | 0,227 | 0,416 |
| **3-butyryl-7- nitrocoumarine** | 0,000 | 0,001 | 0,005 | 0,007 | 0,009 | 0,012 | 0,015 | 0,039 | 0,096 |
| **3-acétyl-4-méthyl-7-nitrocoumarine** | 0,000 | -0,003 | -0,002 | -0,001 | 0,002 | 0,008 | 0,023 | 0,051 | 0,110 |
| **7-nitrocoumarine-3-carboxymorpholide** | 0,000 | -0,002 | -0,001 | 0,001 | 0,005 | 0,014 | 0,037 | 0,068 | 0,109 |
| **Contrôle** | 0,000 | -0,002 | 0,000 | 0,005 | 0,019 | 0,085 | 0,169 | 0,246 | 0,400 |

**Tableau 3 :**

| Valeurs numériques associées à la figure 2 | | | | | | | | | |
|---|---|---|---|---|---|---|---|---|---|
| **Composé\Temps(mn)** | **0** | **30** | **60** | **90** | **120** | **150** | **180** | **210** | **240** |
| **7-nitrocoumarine** | 0 | 77 | 519 | 1447 | 2915 | 4713 | 7160 | 10182 | 14189 |
| **4-méthyl-7-nitrocoumarine** | 0 | 15 | 302 | 974 | 2008 | 3299 | 5097 | 7545 | 11125 |
| **méthyl-7-nitrocoumarine-3-carboxylate** | 0 | 174 | 702 | 1297 | 2035 | 2905 | 3946 | 5005 | 6668 |
| **éthyl-7-nitrocoumarine-3-carboxylate** | 0 | 173 | 814 | 1703 | 2813 | 4126 | 5646 | 7370 | 10859 |
| **acide 7-nitrocoumarine 3-carboxylique** | 0 | -67 | 4 | 181 | 586 | 1694 | 6724 | 17495 | 31314 |
| **3-butyryl-7-nitrocoumarine** | 0 | 64 | 387 | 894 | 1538 | 2344 | 3329 | 4642 | 6845 |
| **3-acétyl-4-méthyl-7-nitrocoumarine** | 0 | 40 | 390 | 992 | 1804 | 2811 | 4090 | 5576 | 8017 |
| **7-nitrocoumarine-3-carboxymorpholide** | 0 | 180 | 665 | 1376 | 2414 | 3796 | 5817 | 8454 | 12263 |
| **Contrôle** | 0 | -214 | -159 | -205 | -174 | -177 | -131 | -104 | -101 |

### B - Etude de la valeur de l'acide 7-nitrocoumarine 3-carboxylique à différentes concentrations pour le contrôle de la croissance de la souche d'Escherichia coli (NCTC 10418) :

### 1°) Méthode :

L'influence de la concentration en acide 7-nitrocoumarine-3-carboxylique sur la sensibilité de la détection de la souche d' *E.coli* NCTC 10418 a été étudiée en faisant varier la gamme de concentration de l'indicateur de 0 à 0,262 mmol/l. Les autres conditions de l'essai étaient les mêmes que celles décrites dans le paragraphe A précédent.

### 2°) Résultats :

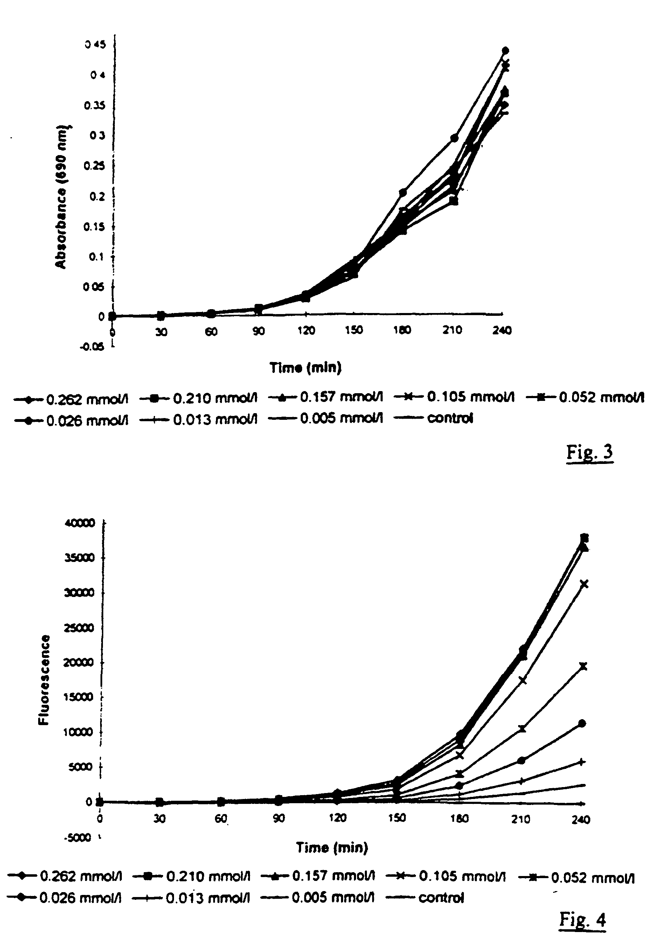

On voit clairement sur la figure 3 que la concentration d'acide 7-nitrocoumarine 3-carboxylique n'a pas d'effet significatif sur la croissance d'*E*. *coli* comme l'indique la superposition des cinétiques obtenues avec les différentes concentrations en substrat et ce même par rapport au contrôle. Le fait que ce composé ne soit pas du tout inhibiteur, est reflété par le taux de fluorescence généré par la nitro-réduction.

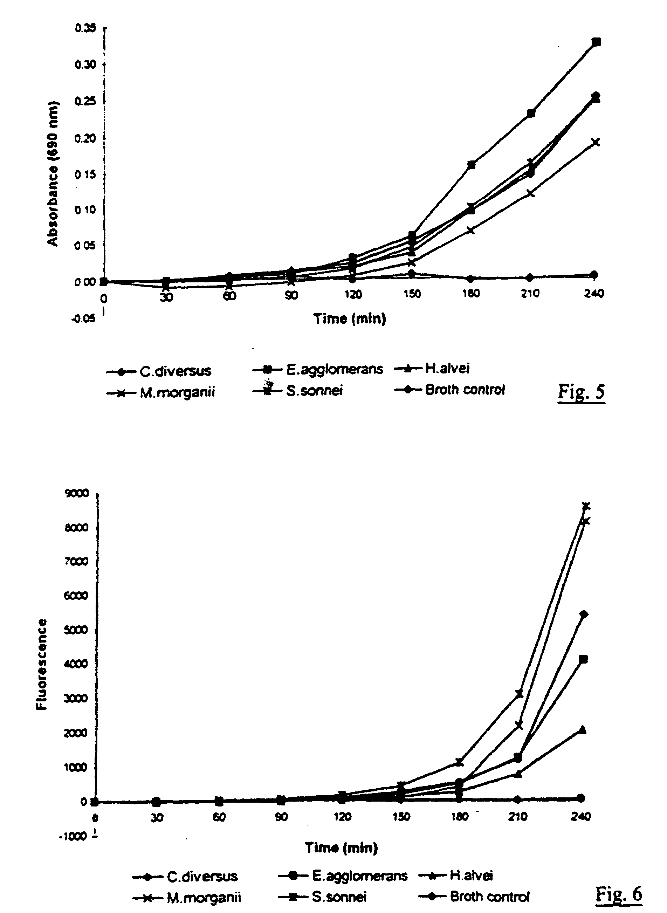

La figure 4 montre que la production de fluorescence augmente avec la concentration de substrat jusqu'à saturation du substrat autour de 0,157 mmol/l (36,9 µg/ml). Au-delà de cette concentration, la sensibilité de la réaction fluorescente n'est plus augmentée.

Pour faciliter la lecture des figures 3 et 4, les deux tableaux 4 et 5 qui suivent énumèrent les valeurs associées à ces figures.

**Tableau 4 :**

| Valeurs numériques associées à la figure 3 | | | | | | | | | |
|---|---|---|---|---|---|---|---|---|---|
| **Composé\Temps(mn)** | **0** | **30** | **60** | **90** | **120** | **150** | **180** | **210** | **240** |
| **0,262 mmol/l** | 0,000 | 0,000 | 0,002 | 0,009 | 0,027 | 0,069 | 0,144 | 0,213 | 0,346 |
| **0,210 mmol/l** | 0,000 | 0,000 | 0,003 | 0,010 | 0,027 | 0,072 | 0,138 | 0,187 | 0,346 |
| **0,157 mmol/l** | 0,000 | -0,001 | 0,002 | 0,009 | 0,027 | 0,073 | 0,151 | 0,205 | 0,372 |
| **0,105 mmol/l** | 0,000 | 0,000 | 0,002 | 0,008 | 0,026 | 0,068 | 0,164 | 0,227 | 0,416 |
| **0,052 mmol/l** | 0,000 | 0,001 | 0,003 | 0,011 | 0,031 | 0,082 | 0,153 | 0,225 | 0,408 |
| **0,026 mmol/l** | 0,000 | 0,001 | 0,004 | 0,011 | 0,032 | 0,079 | 0,201 | 0,290 | 0,436 |
| **0,013 mmol/l** | 0,000 | 0,000 | 0,003 | 0,011 | 0,032 | 0,088 | 0,149 | 0,233 | 0,371 |
| **0,005 mmol/l** | 0,000 | 0,000 | 0,003 | 0,011 | 0,034 | 0,090 | 0,155 | 0,246 | 0,411 |
| **Contrôle** | 0,000 | 0,002 | 0,005 | 0,011 | 0,025 | 0,061 | 0,174 | 0,241 | 0,332 |

**Tableau 5 :**

| Valeurs numériques associées à la figure 4 | | | | | | | | | |
|---|---|---|---|---|---|---|---|---|---|
| **Composé\Temps(mn)** | **0** | **30** | **60** | **90** | **120** | **150** | **180** | **210** | **240** |
| **0,262 mmol/l** | 0 | 3 | 131 | 446 | 1190 | 3037 | 9721 | 22066 | 37949 |
| **0,210 mmol/l** | 0 | -6 | 88 | 354 | 979 | 2588 | 9000 | 21589 | 37823 |
| **0,157 mmol/l** | 0 | -74 | 48 | 280 | 811 | 2231 | 8264 | 21022 | 36517 |
| **0,105 mmol/l** | 0 | -67 | 4 | 181 | 586 | 1694 | 6724 | 17495 | 31314 |
| **0,052 mmol/l** | 0 | -83 | -76 | 33 | 241 | 861 | 4004 | 10584 | 19530 |
| **0,026 mmol/l** | 0 | -91 | -110 | -39 | 71 | 467 | 2311 | 6028 | 11387 |
| **0,013 mmol/l** | 0 | -113 | -141 | -76 | -22 | 174 | 1068 | 3073 | 5902 |
| **0,005 mmol/l** | 0 | -134 | -164 | -122 | -94 | -9 | 406 | 1307 | 2466 |
| **Contrôle** | 0 | -296 | -159 | -168 | -149 | -156 | -131 | -110 | -119 |

### C - Etude de la valeur de l'acide 7-nitrocoumarine 3-carboxylique comme indicateur de croissance de différentes souches d'entérobactéries :

### 1°) Méthode :

Dans cette expérience, il a été vérifié s'il était possible de détecter d'autres souches d'entérobactéries que celle d' *E. coli* à l'aide de l'acide 7-nitrocoumarine-3-carboxylique. Les conditions de cette étude étaient similaires à celles de la première expérience (paragraphe A).

Cinq souches sauvages appartenant aux espèces *Citrobacter diversus, Enterobacter agglomerans, Hafnia alvei, Morganella morganii* et *Shigella sonnei* ont été testées

### 2°) Résultats :

La figure 6 montre la réduction de l'acide 7-nitrocoumarine-3-carboxylique par des souches sauvages appartenant à cinq espèces différentes d'entérobactéries. Toutes les souches testées ont été capables de réduire ce composé. Une comparaison des cinétiques de fluorescence de la figure 6 avec les cinétiques de croissance de la figure 5 montre une excellente corrélation entre croissance et fluorescence, indiquant ainsi que l'acide 7-nitrocoumarine-3-carboxylique est un très bon indicateur de croissance.

Pour faciliter la lecture des figures 5 et 6, les deux tableaux 6 et 7 qui suivent énumèrent les valeurs associées à ces figures.

**Tableau 6 :**

| Valeurs numériques associées à la figure 5 | | | | | | | | | |
|---|---|---|---|---|---|---|---|---|---|
| **Composé\Temps(mn)** | **0** | **30** | **60** | **90** | **120** | **150** | **180** | **210** | **240** |
| ***C. diversus*** | 0,000 | 0,000 | 0,005 | 0,011 | 0,020 | 0,050 | 0,092 | 0,143 | 0,250 |
| ***E. agglomerans*** | 0,000 | -0,002 | 0,002 | 0,006 | 0,027 | 0,057 | 0,155 | 0,225 | 0,323 |
| ***H. alvei*** | 0,000 | -0,001 | 0,004 | 0,009 | 0,016 | 0,034 | 0,092 | 0,149 | 0,246 |
| ***M. morganii*** | 0,000 | -0,010 | -0,010 | -0,005 | 0,003 | 0,020 | 0,064 | 0,116 | 0,187 |
| ***S. sonnei*** | 0,000 | -0,001 | 0,000 | 0,002 | 0,013 | 0,042 | 0,097 | 0,159 | 0,246 |
| **Contrôle** | 0,000 | -,0002 | -0,002 | 0,003 | -0,002 | 0,005 | -0,003 | -0,001 | 0,003 |

**Tableau 7 :**

| Valeurs numériques associées à la figure 6 | | | | | | | | | |
|---|---|---|---|---|---|---|---|---|---|
| **Composé\Temps(mn)** | **0** | **30** | **60** | **90** | **120** | **150** | **180** | **210** | **240** |
| ***C diversus*** | 0 | 3 | 3 | 34 | 92 | 281 | 546 | 1209 | 5393 |
| ***E. agglomerans*** | 0 | -15 | -3 | 9 | 74 | 199 | 501 | 1246 | 4099 |
| ***H. alvei*** | 0 | -24 | -30 | 0 | 40 | 122 | 260 | 775 | 2048 |
| ***M. morganii*** | 0 | -42 | -24 | -15 | 31 | 95 | 403 | 2170 | 8128 |
| ***S. sonnei*** | 0 | -9 | 15 | 52 | 162 | 434 | 1108 | 3095 | 8567 |
| **Contrôle** | 0 | 9 | 0 | 24 | 21 | 34 | 43 | 24 | 61 |

Bien que les résultats ne soient pas présentés ici, il a été vérifié que l'on obtenait le même genre de cinétiques avec la plupart des micro-organismes, autres que les entérobactéries, par exemple les bacilles à Gram négatif non fermentants, les staphylocoques, les streptocoques, les *Listeria* et les levures. Tous étaient capables de réduire les dérivés nitrocoumariniques, en particulier l'acide 7-nitrocoumarine-3-carboxylique, pour donner un signal fluorescent, montrant l'universalité de cette méthode de détection de la croissance, comme cela est illustré par les résultats de l'étude suivante.

### D- Etude de la valeur de l'acide 7-nitrocoumarine-3-carboxylique comme indicateur de croissance pour une large variétés de micro-organismes :

### 1°) Méthode :

Cette étude a été réalisée en utilisant l'acide 7-nitrocoumarine-3-carboxylique et en testant seize (16) souches de micro-organismes, représentant une large variété de bactéries et incluant une levure. La liste des souches étudiées est énumérée dans le tableau 8 défini ci-après.

Un échantillon de 10 mg de l'indicateur est dissout à chaud dans 4 ml d'eau distillée. Cette solution est ensuite ajoutée à 96 ml de bouillon Trypticase-Soja, et le mélange est filtré de manière stérile.

Les souches sont cultivées à 35°C en bouillon Trypticase-Soja pendant 24 h. Chaque culture est alors diluée au 1/1000 dans du bouillon Trypticase-Soja, puis des dilutions successives au 1/10 sont effectuées jusqu'à ne plus avoir de cellules microbiennes dans la prise d'essai.

On réalise ainsi une gamme de dilutions allant de 10⁻³ à 10⁻¹³.

Cinquante microlitres de chaque dilution et cinquante microlitres de solution de bouillon Trypticase-Soja, avec ou sans acide 7-nitrocoumarine-3-carboxylique, sont ajoutés dans les puits d'une plaque de microtitration.

Les plaques ainsi préparées sont mises à incuber à 35°C et lues, d'une part, à instant de départ (T zéro) et, d'autre part, après 24 h :
- en densité optique (690 nm), à l'aide du spectrophotomètre Anthos 2001 (Labtech International Limited), pour les puits ne contenant pas l'acide 7-nitrocoumarine-3-carboxylique, et
- en fluorescence (excitation : 365 nm et émission : 440 nm), à l'aide du fluorimètre Biolite F1 2001 (Labtech International Limited), pour les puits contenant l'acide 7-nitrocoumarine-3-carboxylique.

Afin de vérifier la correspondance entre croissance et fluorescence, ainsi que l'absence de toxicité de l'indicateur, tous les puits ont été repiqués sur gélose Columbia au sang de mouton.

### 2°) Résultats :

L'acide 7-nitrocoumarine-3-carboxylique a été utilisé pour tester seize souches de micro-organismes, représentant une large variété de bactéries et incluant une levure, comme cela est bien indiqué dans le tableau 8 ci-dessous.

Les mesures de fluorescence (première ligne pour chaque souche) et de densité optique (deuxième ligne pour chaque souche) obtenues après 24 h d'incubation sont notées dans le tableau 8. Afin de vérifier la correspondance entre croissance et fluorescence, ainsi que l'absence de toxicité de l'indicateur, tous les puits ont été repiqués sur gélose Columbia au sang de mouton, et ceux qui ont donné des colonies ont été indiqués dans le tableau par des cases en italiques.

Les résultats présentés dans le tableau montrent qu'il existe une très grande corrélation entre la fluorescence due à la réduction de l'indicateur et la croissance microbienne, et cela quel que soit le type de micro-organisme. Les rares différences mineures observées peuvent s'expliquer par de faibles variations possibles au niveau du nombre de cellules vivantes présentes dans les puits des plaques de microtitration, notamment pour les dilutions limites pour lesquelles l'inoculum peut contenir une seule cellule microbienne. La détection de la fluorescence peut donc se substituer à la mise en évidence de la croissance. L'utilisation de l'acide 7-nitrocoumarine-3-carboxylique comme indicateur fluorescent de croissance apparaît extrêmement sensible puisqu'il est possible de détecter un très petit nombre de micro-organismes, qui peut être estimé à une seule cellule vivante, lorsque l'on utilise la méthode des dilutions limites pratiquée lors de cette expérimentation.

Il est à noter que dans cet essai, la souche de levure testée (*Candida albicans*) a généré un faible niveau de fluorescence, comparativement aux bactéries. Cela est dû en fait à l'utilisation du bouillon Tryptycase-Soja qui n'est pas un milieu bien adapté à la croissance des levures. Bien que les résultats ne soient pas présentés, si le milieu Tryptycase-Soja est remplacé par un bouillon RPMI recommandé pour les levures, le niveau de fluorescence obtenu est alors comparable à celui observé avec les bactéries.

Les résultats sont présentés sur le tableau 8 ci-après. Le nombre de dilutions étant important (onze dilutions entre 10⁻³ et 10⁻¹³ plus une douzième valeur concernant un « blanc » faisant office de témoin), et afin de faciliter la compréhension et la lecture de ce tableau, celui-ci a été divisé en deux parties. Une première partie concerne les dilutions comprises entre 10⁻³ et 10⁻⁹. Une seconde partie concerne les dilutions comprises entre 10⁻¹⁰ et 10⁻¹³ et le témoin.

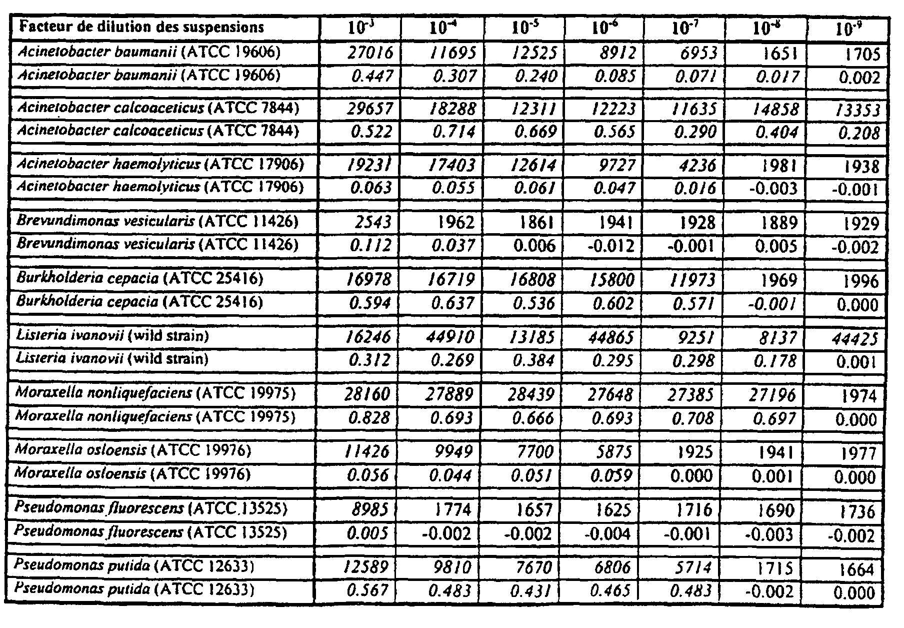

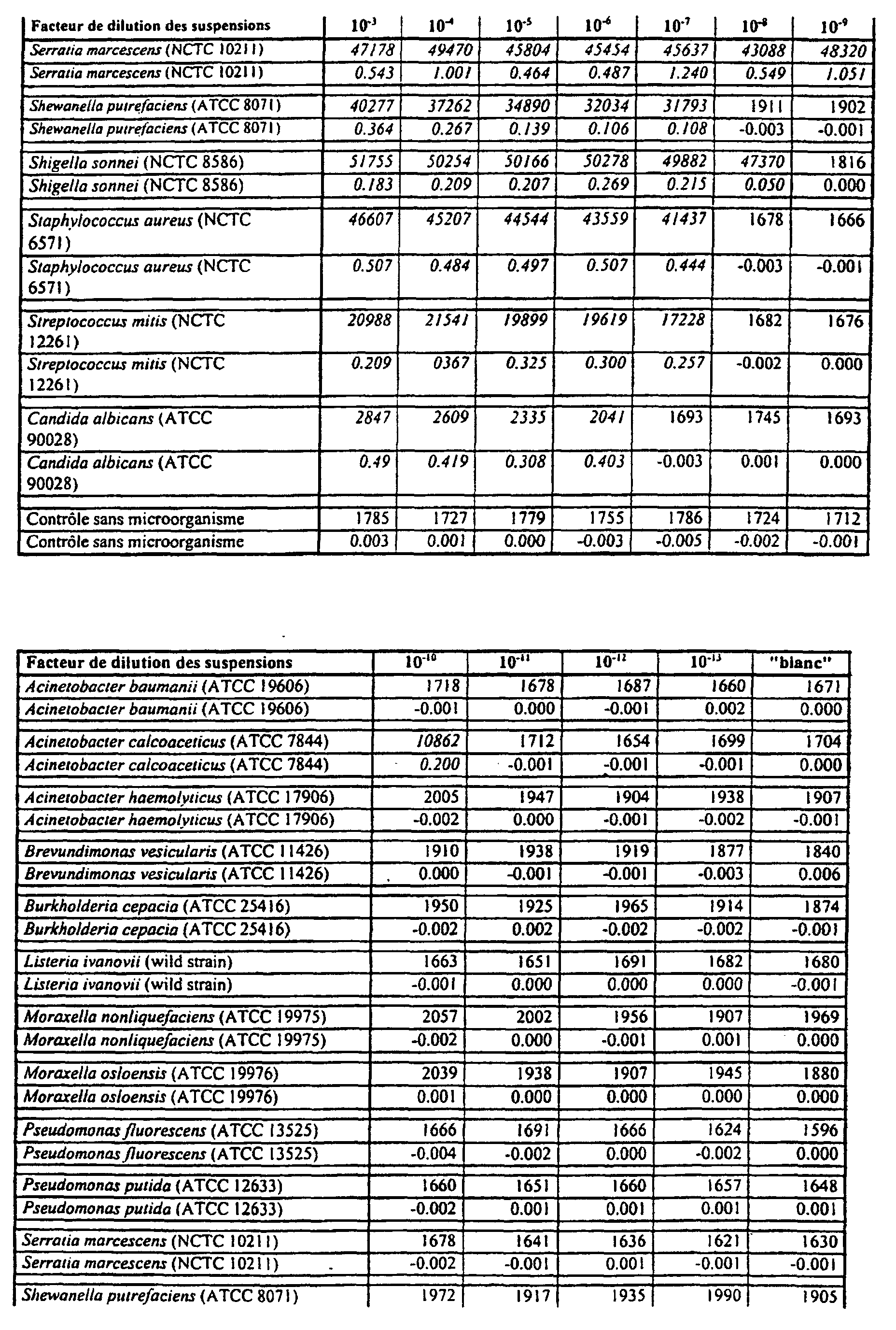

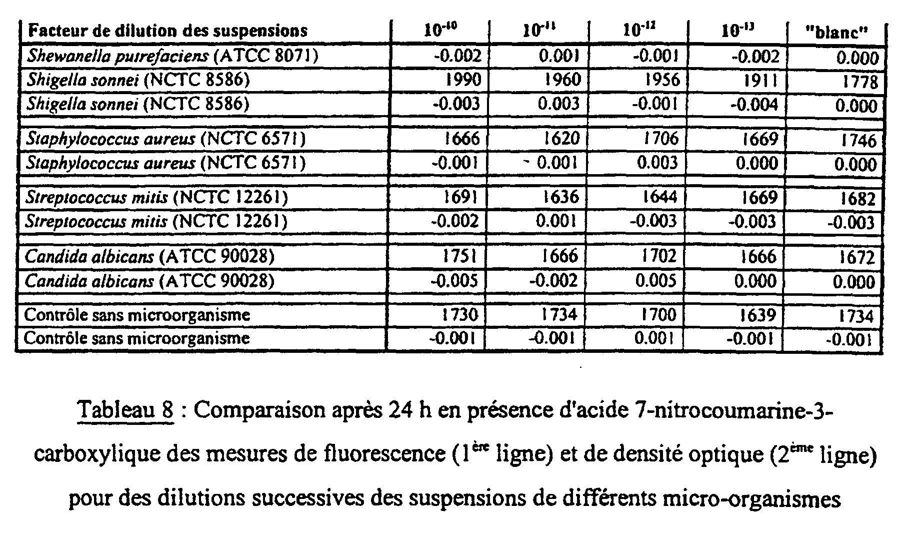

### 3°) Conclusion :

Les dérivés de la 7-nitrocoumarine, et notamment l'acide 7-nitrocoumarine-3-carboxylique, constituent une famille d'indicateurs fluorescents universels de la croissance des micro-organismes.

Les applications de ces nouveaux indicateurs sont nombreuses en microbiologie et concernent toutes les méthodes faisant appel à la détection de la croissance microbienne. Parmi ces applications, on peut citer :
- les méthodes d'antibiogramme et d'antifongigramme (recherche de la sensibilité des micro-organismes aux antibiotiques et antifongiques),
- les méthodes d'identification faisant appel à des tests d'assimilation (mise en évidence de la croissance en présence de substrats utilisés comme seules sources de carbone),
- la mise en évidence de la stérilité d'un échantillon (absence de micro-organismes),
- toute détection de la présence de micro-organismes dans des échantillons susceptibles de les contenir, qu'ils soient cliniques, industriels (alimentaires, pharmaceutiques, cosmétiques...) ou de l'environnement, et
- tout dénombrement de micro-organismes dans un échantillon, en particulier les numérations basées sur la détermination de nombre le plus probable (NPP), bien connue dans le domaine de l'agro-alimentaire.

## Revendications

1. Utilisation d'un composé dans un test de détection et/ou de diagnostic de la présence ou de l'absence de micro-organismes, dans lequel le composé estconstitué par une molécule de 7-nitrocoumarine ou un de ses dérivés.

2. Utilisation, selon la revendication 1, **caractérisée par le fait que** le composé est constitué par une molécule ayant la formule générale suivante : 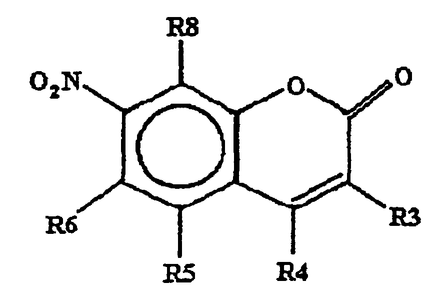 dans laquelle R₃ est choisi parmi : H ou COZ, où Z permet la formation d'une cétone, d'un acide, d'un ester ou de tout autre composé aliphatique,
dans laquelle R₄ est choisi parmi : H ou trifluorométhyle (CF₃) ou tout composé aliphatique.

3. Utilisation, selon la revendication 2, **caractérisée par le fait que** R₃ est constitué par : COOCH₃, COOC₂H₅, COOH, COC₃H₇, CONC₄H₄O ou COCH₃, et R₄ est constitué par : H ou CH₃.

4. Utilisation, selon l'une quelconque des revendications 1 à 3, **caractérisée par le fait qu'**il est constitué par de l'acide 7-nitrocoumarine-3-carboxylique.

5. Utilisation, selon la revendication 4, **caractérisée par le fait que** la concentration en acide 7-nitrocoumarine-3-carboxylique est comprise entre 0,05 et 0,3 mmol/l.

6. Utilisation d'un composé, selon l'une quelconque des revendications 1 à 5, pour permettre la détection de la croissance microbienne afin d'effectuer un contrôle de stérilité.

7. Utilisation d'un composé, selon l'une quelconque des revendications 1 à 5, pour permettre la détection de la croissance microbienne afin d'effectuer une numération des micro-organismes présents dans l'échantillon.

8. Utilisation d'un composé, selon l'une quelconque des revendications 1 à 5, pour permettre la détection de la croissance microbienne afin de déterminer la sensibilité d'un micro-organisme aux agents antimicrobiens.

9. Utilisation d'un composé, selon l'une quelconque des revendications 1 à 5, pour permettre la détection de la croissance microbienne afin de détecter la présence d'au moins un micro-organisme.

10. Procédé de détection d'un micro-organisme ou d'un groupe de micro-organismes dans un échantillon susceptible de les contenir, comprenant les étapes consistant à :
- ajouter à un milieu de culture, contenant l'échantillon, au moins un composé, à base de 7-nitrocoumarine ou un de ses dérivés, et
- rechercher la formation d'un produit fluorescent, la présence ou l'absence de cette fluorescence permet de conclure respectivement à la présence ou à l'absence du micro-organisme ou du groupe de micro-organismes recherché.

11. Procédé d'identification d'au moins un micro-organisme dans un échantillon susceptible de les contenir comprenant les étapes suivantes :
- ajouter dans différents puits d'identification un milieu de culture, contenant chacun une seule source de carbone, une fraction de l'échantillon à analyser et au moins un composé, à base de 7-nitrocoumarine ou un de ses dérivés, et
- rechercher la formation d'un produit fluorescent, dans chaque puits, la présence ou l'absence de cette fluorescence sur l'ensemble des puits permet d'identifier le micro-organisme.

## Claims

1. The use of a compound in a test for detecting and/or diagnosing the presence or absence of microorganisms, wherein the compound is made up of a molecule of 7-nitrocoumarin or one of its derivatives.

2. The use, according to Claim 1, **characterised in that** the compound is made up of a molecule having the following general formula: 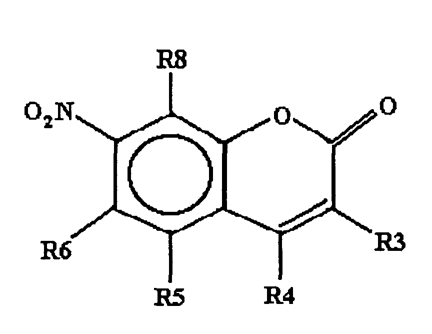 wherein R₃ is either: H or COZ, where Z enables the formation of a ketone, an acid, an ester or any other aliphatic compound,
wherein R₄ is either: H or trifluoromethyl (CF₃) or any other aliphatic compound.

3. The use, according to Claim 2, **characterised in that** R₃ is made up of: COOCH₃, COOC₂H₅, COOH, COC₃H₇, CONC₄H₄O or COCH₃, and R₄ is made up of: H or CH₃.

4. The use, according to any one of Claims 1 to 3, **characterised in that** it is made up of 7-nitrocoumarin-3-carboxylic acid.

5. The use, according to Claim 4, **characterised in that** the concentration of 7-nitrocoumarin-3-carboxylic acid is between 0.05 and 0.3 mmol/l.

6. The use of a compound, according to any one of Claims 1 to 5, to enable the detection of microbial growth in order to carry out a sterility check.

7. The use of a compound, according to any one of Claims 1 to 5, to enable the detection of microbial growth in order to perform a count of the microorganisms present in the sample.

8. The use of a compound, according to any one of Claims 1 to 5, to enable the detection of microbial growth in order to determine the sensitivity of a microorganism to antimicrobial agents.

9. The use of a compound, according to any one of Claims 1 to 5, to enable the detection of microbial growth in order to detect the presence of at least one microorganism.

10. A method for detecting one microorganism or a group of microorganisms in a sample which may contain them, including the steps of:
- adding to a culture medium, containing the sample, at least one compound with a 7-nitrocoumarin base or a derivative thereof, and
- searching for the formation of a fluorescent product, the presence or absence of this fluorescence enabling a conclusion to be reached as to the presence or the absence, respectively, of the sought microorganism or group of microorganisms.

11. The method for identifying at least one microorganism in a sample which may contain them including the following steps:
- adding in various identification wells a culture medium, each of which contains a single carbon source, a fraction of the sample to be analysed and at least one compound, with a 7-nitrocoumarin base or a derivative thereof, and
- searching for the formation of a fluorescent product in each well, the presence or absence of this fluorescence over all the wells enabling the microorganism to be identified.

## Patentansprüche

1. Die Verwendung einer Verbindung bei einem Test zur Ermittlung und/oder Diagnose der Anwesenheit oder Abwesenheit von Mikroorganismen, wobei die Verbindung aus einem Molekül 7-Nitrocumarin oder einem seiner Derivate zusammengesetzt ist.

2. Verwendung gemäß Anspruch 1, **dadurch gekennzeichnet, dass** die Verbindung aus einem Molekül mit der folgenden allgemeinen Formel zusammengesetzt ist: 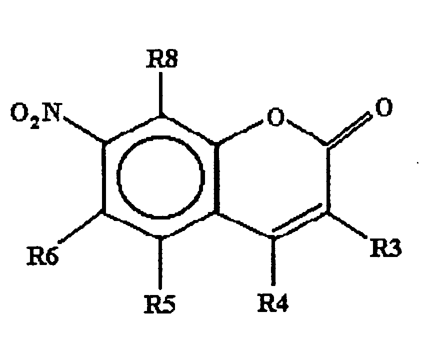 wobei R₃ aus H oder COZ ausgewählt ist, wo Z die Bildung eines Ketons, einer Säure, eines Esters oder einer anderen beliebigen aliphatischen Verbindung ermöglicht,
wobei R₄ aus H oder Trifluoromethyl (CF₃) oder einer anderen beliebigen aliphatischen Verbindung ausgewählt ist.

3. Verwendung gemäß Anspruch 2, **dadurch gekennzeichnet, dass** R₃ aus COOCH₃, COOC₂H₅, COOH, COC₃H₇, CONC₄H₄O oder COCH₃ und R₄ aus H oder CH₃ zusammengesetzt ist.

4. Verwendung gemäß einem der Ansprüche 1 bis 3, **dadurch gekennzeichnet, dass** sie aus 7-Nitrocumarin-3-carboxyl zusammengesetzt ist.

5. Verwendung gemäß Anspruch 4, **dadurch gekennzeichnet, dass** die Konzentration von 7-Nitrocumarin-3-carboxyl zwischen 0,05 und 0,3 mmol/l liegt.

6. Verwendung einer Verbindung gemäß einem der Ansprüche 1 bis 5, um das Ermitteln des mikrobiellen Wachstums zu ermöglichen, um eine Sterilitätsprüfung durchzuführen.

7. Verwendung einer Verbindung gemäß einem der Ansprüche 1 bis 5, um das Ermitteln des mikrobiellen Wachstums zu ermöglichen, um die in der Probe anwesenden Mikroorganismen zu zählen.

8. Verwendung einer Verbindung gemäß einem der Ansprüche 1 bis 5, um das Ermitteln des mikrobiellen Wachstums zu ermöglichen, um die Suszeptibilität eines Mikoorganismus gegenüber antimikrobiellen Agenzien zu bestimmen.

9. Verwendung einer Verbindung gemäß einem der Ansprüche 1 bis 5, um das Ermitteln des mikrobiellen Wachstums zu ermöglichen, um die Anwesenheit von mindestens einem Mikroorganismus zu ermitteln.

10. Ein Verfahren zum Ermitteln eines Mikroorganismus oder einer Gruppe von Mikroorganismen in einer Probe, die diese enthalten kann, das die folgenden Schritte umfasst:
- Zugeben von mindestens einer auf 7-Nitrocumarin oder einem seiner Derivate basierenden Verbindung zu einem die Probe enthaltenden Nährmedium, und
- Suchen nach der Bildung eines fluoreszierenden Produkts, wobei anhand der Anwesenheit oder Abwesenheit dieser Fluoreszenz entschieden werden kann, ob der gesuchte Organismus oder die gesuchte Gruppe von Mikroorganismen anwesend oder abwesend ist.

11. Ein Verfahren zum Identifizieren von mindestens einem Mikroorganismus in einer Probe, die diese enthalten kann, das die folgenden Schritte umfasst:
- Zugeben von einem Nährmedium, das je eine einzelne Kohlenstoffquelle, eine zu analysierende Probenfraktion und mindestens eine auf 7-Nitrocumarin oder einer seiner Derivate basierende Verbindung enthält, in unterschiedliche Identifikations-Löcher, und
- Suchen nach der Bildung eines fluoreszierenden Produkts in jedem Loch, wobei die Anwesenheit oder Abwesenheit dieser Fluoreszenz in allen Löchern die Identifikation des Mikroorganismus ermöglicht.
